(19) Europäisches Patentamt
European Patent Office
Office européen des brevets

(11) Publication number: 0 301 706 A2

(12) # EUROPEAN PATENT APPLICATION

(21) Application number: 88305981.8

(22) Date of filing: 29.06.88

(51) Int. Cl.[4]: C08G 69/00 , C08G 59/54

(30) Priority: 27.07.87 US 78323
27.07.87 US 78310

(43) Date of publication of application:
01.02.89 Bulletin 89/05

(84) Designated Contracting States:
DE FR GB NL SE

(71) Applicant: TEXACO DEVELOPMENT CORPORATION
2000 Westchester Avenue
White Plains New York 10650(US)

(72) Inventor: Waddill, Harold George
7108 Geneva Circle
Austin Texas 78723(US)
Inventor: Lin, Jiang-Jen
2617 Oak Meadow Drive
Round Rock Texas 78664(US)
Inventor: Speranza, George Phillip
2800 Silverleaf Circle
Austin Texas 78757(US)

(74) Representative: Harrison, Michael Robert et al
Urquhart-Dykes & Lord 91 Wimpole Street
London W1M 8AH(GB)

(54) **Aromatic amidoamines.**

(57) Amidoamine reaction products are formed by reaction of an aromatic di- or tricarboxylic acid, ester or anhydride with a polyetheramine.

EP 0 301 706 A2

# AROMATIC AMIDOAMIDES

## BACKGROUND OF THE INVENTION

### Technical Field of the Invention

This invention relates to aromatic amidoamines. More particularly, this invention relates to novel aromatic amidoamines derived from polyoxyalkylene polyamines and aromatic dicarboxylic or tricarboxylic acids, esters or anhydrides thereof.

In one embodiment, this invention relates to novel aromatic amidoamines prepared by reacting at least about 2 mole equivalents of a polyoxyalkylene diamine or polyoxyalkylene triamine with an aromatic dicarboxylic or tricarboxylic acid to thereby couple each of the carboxyl groups to an amine group by the formation of an amide linkage therebetween. The reaction is preferably conducted at autogenous pressure at a temperature within the range of about 150° to about 250° C.

These aromatic amidoamines of the present invention are liquids or amorphous solids, depending upon the starting materials, and can be used as raw materials for a wide variety of purposes such as, for example, as chain extenders for epoxy resins, curing agents for epoxy resins, as raw materials for the manufacture of polyureas, thickening agents, etc. The products may also be used as raw materials for the preparation of fuel and lubricant additives, for textile and fiber treating agents, for the preparation of adhesives, for use in the manufacture of polyureas, for use in encapsulation and moulding applications, etc.

In another embodiment, the amidoamines are prepared by reacting at least about 2 mole equivalents of a polyether amine with an aromatic dicarboxylic acid to thereby couple each of the carboxyl groups to an amine group by formation of an amide linkage therebetween. The reaction to produce the amidoamines is preferably conducted at autogenous pressure at a temperature within the range of about 0° C to about 250° C and preferably above 150° C. These amidoamines are expecially useful as curatives for epoxy resins. They exhibit improved properties which allow for a rapid curing system, even under ambient conditions. Excellent properties are exhibited on curing epoxy resins with these products, and materials are provided which should be useful in a number of applications such as coatings, adhesives, encapsulations, laminates and composite fabrications and sealants.

The present invention provides an amidoamine reaction product formed by reaction of an aromatic di- or tricarboxylic acid, ester or anhydride with a polyetheramine.

In one embodiment, the amidoamine is formed by reacting the polyoxypropylene and/or polyoxyethylene diamine or triamine with an aromatic dicarboxylic acid or tricarboxylic acid in molar portions such that at least about 2 mole equivalents of amine are provided in the reaction mixture for each mole equivalent of carboxylic acid present in the reaction mixture.

When the amine is in excess, a primary amine group of the polyamine will preferentially react with each of the carboxyl groups of the aromatic di- or tricarboxylic acid, ester ot anhydride thereof and be linked thereto through an amide linkage. The product is substantially free from terminal carboxylic acid groups and contains primarily terminal primary amine groups.

The reaction between the polyoxyalkylene polyamine and the aromatic carboxylic acid, ester or anhydride thereof, is preferably conducted in an autoclave at autogenous pressure which may suitable range from about 0 to about 1 atmospheres and at a temperature of from about 150° to about 250° C. The reaction time required for completion of the reaction will normally range from about 0.5 to about 12 hours. By-product water of reaction is preferably removed as formed, so that the reaction product obtained at the end of the reaction is the desired final product.

In another embodiment, the present invention provides condensation products of polycarboxylic acids and polyetheramines and their use as curatives of epoxy resins. These amidoamines, when combined with an epoxy resin in the proper stoichiometric amount, result in a rapid curing system, even under ambient conditions.

The condensation products are the product of a reaction between certain polyetheramines, exemplified by JAFFAMINE® EDR series and certain dicarboxylic acids at a temperature of 150° C to 250° C and atmospheric pressure.

One of these products can be represented by formula I:

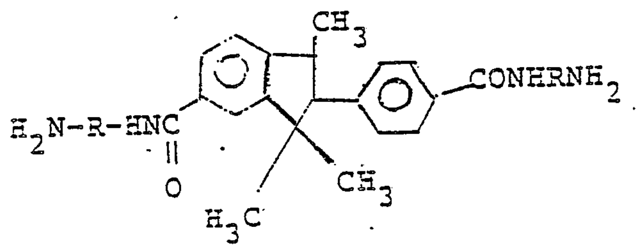

(I)

wherein R = polyether segment, either polyoxypropylene or polyoxyethylene or both in combination.

Another of these amidoamine products may be represented by formula II:

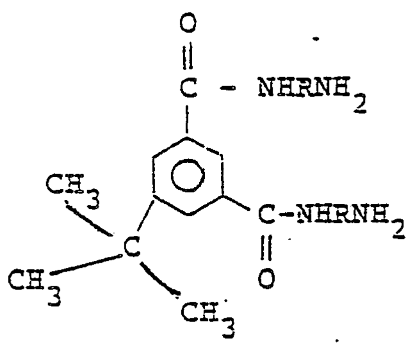

where R = a polyether segment, either polyoxypropylene or polyoxyethylene or both in combination.

## Detailed Description of one Embodiment

The amidoamine reaction products of the present invention are reaction products which contain as the principle reaction component, an amidoamine having the formula:

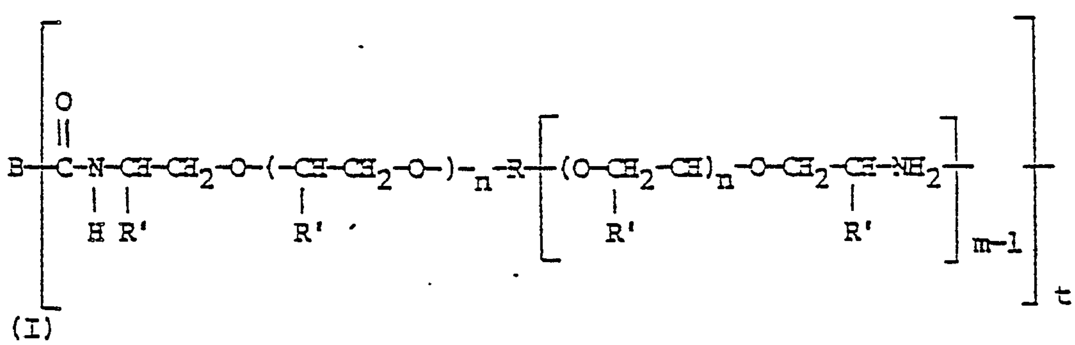

(I)

wherein B is the nucleus of an aromatic dicarboxylic acid or tricarboxylic acid containing from about 6 to about 18 carbon atoms, and R is the nucleus of an oxyalkylation susceptible dihydric or trihydric alcohol containing 2 to 6 carbon atoms, m is an integer having a value of 2 to 3, t is an integer having a value of from 2 to 3 and n is a number having an average value of from about 0 to about 50, and R′ represents hydrogen or methyl.

Encompassed within the above formula are reaction products of aromatic dicarboxylic acids with polyoxyalkylene diamines which will contain two terminal primary amine groups, reaction products or aromatic tricarboxylic acids with polyoxypropylene diamines which will contain three terminal primary amine groups, reaction products of aromatic dicarboxylic acids with polyoxypropylene triamines which will contain an average of about 4 terminal primary amine groups and reaction products of aromatic tricarboxylic acids with polyoxypropylene triamines which will contain an average of about 6 terminal primary amine groups.

By using mixtures of aromatic dicarboxylic acids with aromatic tricarboxylic acids or mixtures of

polyoxyalkylene diamines with polyoxyalkylene triamines, reaction products can be provided having a primary amine functionality intermediate 2 to about 6.

## The Carboxylic Acid Starting Material

The aromatic dicarboxylic acid or tricarboxylic acid starting material for the present invention may be any suitable aromatic carboxylic acid having the desired functionality and containing a total of from about 8 to about 20 carbon atoms. The anhydrides and lower alkyl esters of the acids wherein the alkyl group contains 1 to 4 carbon atoms, and is preferably methyl, may also be used.

Examples of aromatic dicarboxylic acid and tricarboxylic acids that may be used as starting materials for the present invention include acids such as terephthalic acid, isophthalic acid, trimesic acid, 1,1,3-trimethyl-3-phenylidan-4′,5-dicarboxylic acid, 2,6-naphthalene dicarboxylic acid, t-butyl isophthalic acid, etc. (i.e., benzene dicarboxylic acids and tricarboxylic acids, hemimellitic acid, trimellitic acid, 2-phenyl pentanedioic acid, phenyl succinic acid, etc.).

## The Polyoxyalkylene Polyamine Starting Materials

The polyoxyalkylene polyamine starting materials for the present invention include polyoxypropylene diamines and triamines, polyoxyethylene diamines, and polyoxyalkylene diamines and triamines containing mixtures of both ethylene oxide and propylene oxide and, preferably, mixtures of from about 5 to about 40 wt.% of ethylene oxide with, correspondingly, from about 95 to 60 wt.% of propylene oxide. Where mixed propylene oxide/ethylene oxide polyols are employed, the ethylene oxide and propylene oxide may be premixed prior to reaction to form a hetero copolymer, or the ethylene oxide and the propylene oxide may be sequentially added to the ethoxylation kettle to form block oxypropylene/oxyethylene copolymers.

In general, the polyoxyalkylene polyamine starting material may be defined as a polyoxylalkylene polyamine having the formula:

$$\text{(II)} \qquad R-\left[(OCH_2\underset{\displaystyle R'}{\underset{|}{C}H})_n-OCH_2-\underset{\displaystyle R'}{\underset{|}{C}H}-NH_2\right]_m$$

Wherein R is the nucleus of an oxyalkylation-susceptible polyhydric alcohol containing 2 to 12 carbon atoms and 2 and 3 hydroxy groups, and R′ is hydrogen or methyl, n is a number having an average value of 0 to 50, and m is an integer having a value of 2 to 3.

In general, the average molecular weight of the polyoxypropylene diamine or triamine starting material will be from about 200 to about 5,000.

An advantage is obtained when using lower molecular weight diamine and triamine starting materials such as those having average molecular weights of about 200 to about 3,000 in that the final products will have primary amine functionalities and higher molecular weights but will not contain contaminants of the type formed by the oxypropylation of dihydric and trihydric alcohols with propylene oxide to form polyoxypropylene diols and triols having molecular weights of 3,000 to 5,000.

One group of appropriate polyoxyalkylene diamines that may be used are those that are sold by the Texaco Chemical Company as Jeffamine® D-series products having the formula:

$$\text{(III)} \qquad H_2N-\underset{\displaystyle R'}{\underset{|}{C}H}-CH_2-\left[O-CH_2-\underset{\displaystyle R'}{\underset{|}{C}H}\right]_x-NH_2$$

Wherein R′ independently represents hydrogen or methyl and x is a number having an average value of about 1 to about 60.

Representative products having this structural formula include polyoxypropylene diols (wherein R′ is methyl) having an average molecular weight of about 230 wherein the value of x is between 2 and 3 (Jeffamine® D-230 amine), polyoxypropylene diols having an average molecular weight of about 400 wherein x has a value between about 5 and 6 (Jeffamine® D-400 amine), and a polyoxypropylene diol product having an average molecular weight of about 2,000 wherein x has a value of about 33 (Jeffamine® D-2000 amine) and a product having an average molecular weight of about 4,000 wherein x has a value of about 60 (Jeffamine® D-4005 amine).

Another appropriate class of polyoxyalkylene diamines, containing both ethylene oxide and propylene oxide, which may be used are polyoxypropylene diamines that are sold by the Texaco Chemical Company as Jeffamine® ED-series products having the formula:

(IV) 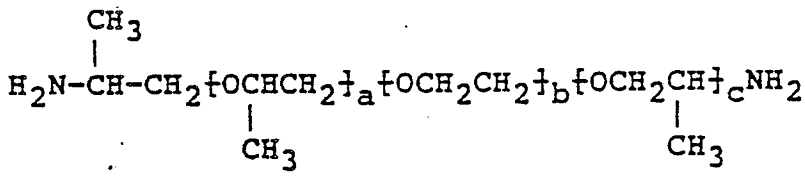

Wherein a + c equals a number having a value of from about 2 to about 10 and b is a number having a value of from about 1 to about 50.

Examples of products having this general formula include a commercial product having an average molecular weight of about 600 where the value of b is about 8.5 and the value of a + c is about 2.5 (Jeffamine® ED-600), a commercial product having an average molecular weight of about 900 wherein the value of a + c is again about 2.5, but the value of b is about 15.5 (Jeffamine® ED-900). Other examples are those wherein a + c has a value of about 2.5 including a product having an average molecular weight of about 2,000 wherein the value of b is about 40 (Jeffamine® ED-2001) and a product having an average molecular weight of about 4,000 wherein the value of b is about 85 (Jeffamine® ED-4000).

An example of appropriate polyoxypropylene triamines that may be used as a starting material for the present invention include triamines sold by Texaco Chemical Company as Jeffamine® T-series products having the formula:

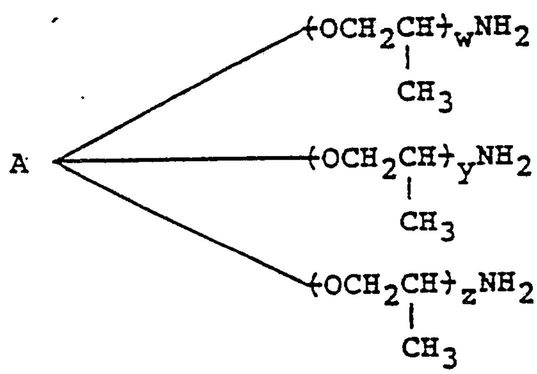

(V)

Wherein A represents the nucleus of an oxyalkylation susceptible trihydric alcohol containing about 3 to about 6 carbon atoms, w, y and z are numbers and the average value of the sum of w + y + z is from about 4 to about 100.

An example of such a product is a commercial product having an average molecular weight of about 400 wherein A represents a trimethylol propane nucleus (Jeffamine® T-403 amine) and a product having an average molecular weight of about 5,000 wherein A represents a glycerol nucleus and the product contains about 85 moles of propylene oxide (Jeffamine® T-5000). The 400 molecular weight product will contain about 5 to about 6 moles of propylene oxide.

Preparation of the Amidoamines

It has been discovered in accordance with the present invention that an amidoamine addition product is preferentially formed when an aromatic dicarboxylic or tricarboxylic acid or ester or anhydride thereof is reacted with an excess of a polyoxyalkylene diamine or triamine at autogenous pressure at a temperature within the range of about 150° to about 250° C for a reaction time within the range of about 0.5 to about 12 hours. Normally, the reaction will go to completion after a reaction time within the range of about 2 to about 6 hours.

By-product water of reaction is preferably removed from the reaction mixture as formed. The reaction is complete when essentially all of the carboxylate groups have reacted with primary amine groups of the polyoxyalkylene diamine or triamine. Under the noncatalytic reaction conditions employed herein, the primary amine groups of the polyoxyalkylene diamine or triamine are essentially unreactive with each other.

The amidoamines that are formed by the process of the present invention are liquid or amorphous solid materials having a molecular weight within the range of about 400 to about 15,000 and containing from about 2 to about 6 terminal primary amine groups, depending on the functionality of the starting materials.

The reaction mixture will comprise an amidoamine addition product which may be generally characterized by the following formula:

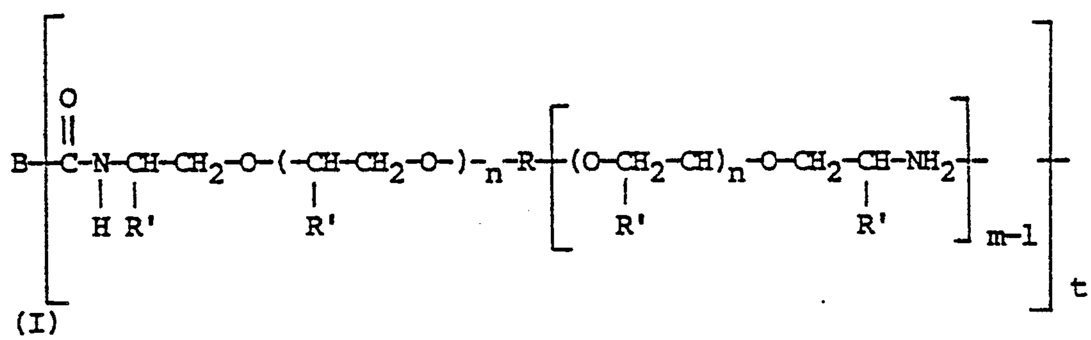

(I)

Wherein B is the nucleus of an aromatic dicarboxylic acid or tricarboxylic acid containing about 8 to about 20 carbon atoms, R is the nucleus of an oxyalkylation-susceptible polyhydric alcohol containing 2 to 12 carbon atoms, R′ is hydrogen or methyl, m is an integer having a value of 2 to 3, n is a number having an average value of about 5 to 50 and t is an integer having a value of 2 to 3.

A variety of molecular configurations are possible for the amidoamine of the present invention, depending on the starting materials.

Thus, an aromatic carboxylic acid such as a tricarboxylic acid may also be reacted with a polyoxyethylene diamine to provide an addition product containing three primary amine groups, as illustrated by the following formula:

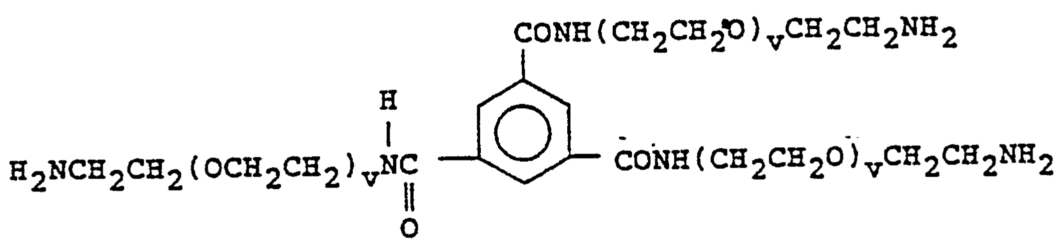

(VI)

Wherein v is a number having a value of about 2-50.

However, if an alkyl substituted aromatic carboxylic acid is used as a starting material, such as tertiary butyl isophthalic acid, an amidoamine addition product will be formed containing 2 or 4 primary amine groups, depending upon whether or not a polyoxypropylene diamine or triamine is used as a starting material, as illustrated by the following formula:

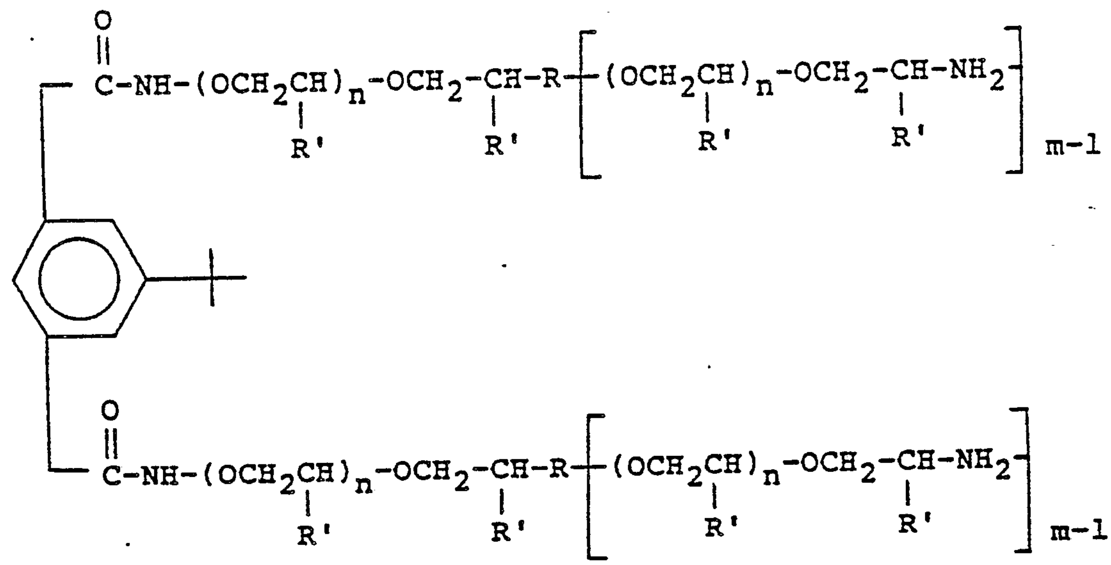

(VII)

Wherein R is the nucleus of an oxyalkylation-susceptible polyhydric alcohol containing 2 to 12 carbon atoms and 2 to 3 hydroxy groups, R′ is hydrogen or methyl, m is an integer having a value of 2 to 3, and n is a number having an average value of 0 to 50.

As another example, if the aromatic dicarboxylic acid that is used as a starting material is a condensed ring aromatic dicarboxylic acid such as 2,6-naphthalene dicarboxylic acid, the resultant addition product will be an amidoamine having the formula:

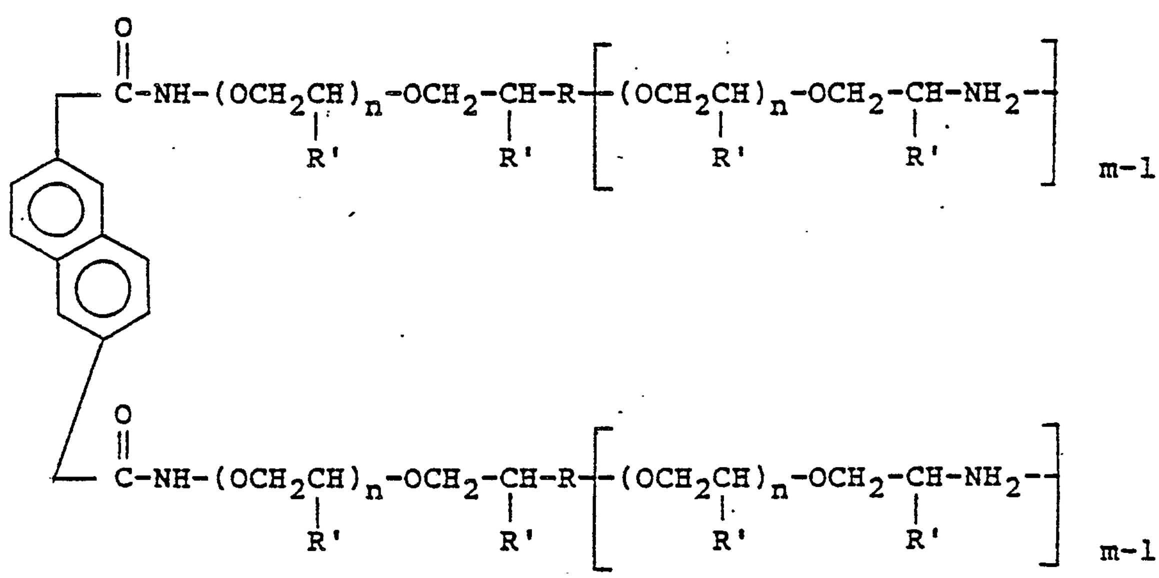

(VIII)

Wherein R is the nucleus of an oxyalkylation-susceptible polyhydric alcohol containing 2 to 12 carbon atoms and 2 to 3 hydroxyl groups, R′ is hydrogen or methyl, m is an integer having a value of 2 to 3, and n is a number having an average value of 0 to 50.

As yet another example, if the starting material that is used is a more complex dicarboxylic acid such as 1,1,3-trimethyl-3-phenylindan-4′,5-dicarboxylic acid, the resultant amidoamine reaction product that is formed can be an amidoamine having the formula:

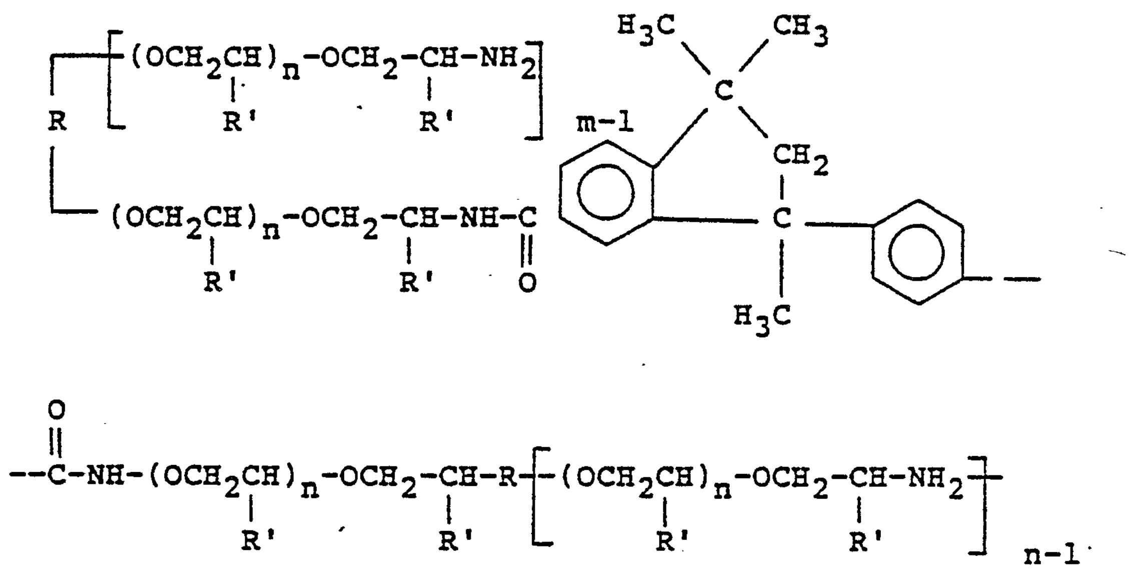

(IX)

Wherein R is the nucleus of an oxyalkylation-susceptible polyhydric alcohol containing 2 to 12 carbon atoms and 2 to 3 hydroxyl groups, R′ is hydrogen or methyl, m is an integer having a value of 2 to 3, and n is a number having an average value of 0 to 50.

## SPECIFIC EXAMPLES

### Group I - Amidoamine Addition Products of a Dibasic Acid with a Polyoxyethylene Diamine

A series of compounds were synthesized using terephthalic and isophthalic acids. Their properties are recorded in the attached table. Some highlights are cited below:

1. Most of these compounds are water and methanol soluble.
2. Most of these compounds are solid with varying melting points up to 150° C. However, some amines from isophthalic acid are liquid.
3. The product from terephthalic acid and BAEE (Bis-aminoethyl ether) cannot be prepared under 200° C, since this salt melts at >200° C.
4. IR, amine and acidity analyzed support the structure of amidoamines.

### Example 1 (6103-99)

To a 100-ml 3-necked flask equipped with thermometer, stirrer, Dean-Stark trap and N2-flow line, was charged 24.9 g of terephthalic acid (0.15 mole) and Jeffamine® EDR-148 amine (44.4g, 0.30 mole), heated to 200° C and kept at this temperature for about 3 hours. During the process, 5.0 cc of water was removed (theoretically 5.4g). After cooling to room temperature, a white solid (mp 95-102° C) was recovered (60.2g). The amine analysis of this product indicated 4.34 meq/g (theoretical 4.55 meq/g). This material is soluble in water or methanol. IR showed the presence of amide. The results of other examples are summarized in the attached table.

TABLE I

| BIS-(AMIDOAMINE) SYNTHESIS - REACTIONS OF POLYOXYETHYLENE DIAMINES WITH DIBASIC ACID AT 2:1 MOLAR RATIO | | | |
|---|---|---|---|
| | BAEE | EDR-148[1] | EDR-192[2] |
| Terephthalic Acid | 6152-19 Solid salt at 200 °C Product cannot be made | 6103-99 mp ca. 140 °C Pale-yellow solid Soluble in $H_2O$, and MeOH IR : Amide | 6152-11 mp 75-82 °C Tan-colored, opaque solid Amine 3.40 meq/g (Theor. 3.9) Soluble in $H_2O$ and MeOH |
| Isophthalic Acid | 6152-20 mp ca. 80 °C Hard orange solid Soluble in $H_2O$ and MeoH | 6125-24 Brown liquid Amine 4.80 meq/g (Theor. 4.7) Acidity 0.29 meq/g Soluble in $H_2O$ and MeOH IR : Amide | 6152-23 Yellow liquid Amine 3.85 meq/g (Theor. 3.87) Acidity 0.18 meq/g Soluble in $H_2O$ and MeOH IR : Amide |

1. Jeffamine® EDR-148 is an amine terminated triethylene glycol having the formula: $H_2N-CH_2CH_2-O-CH_2CH_2-O-CH_2CH_2-NH_2$
2. Jeffamine® EDR-192 is an amine terminated tetraethylene glycol having the formula: $H_2N-CH_2CH_2-O-CH_2CH_2-O-CH_2CH_2-O-CH_2CH$>¥$-NH_2$

<u>High Molecular Weight Tetraamines from Polyoxypropylene Triamines and Dibasic Acids</u>

A process for preparing a series of tetramines via coupling reaction of Jeffamine T-403 or T-5000 (polyoxypropylene triamines manufactured and sold by the Texaco Chemical Company) with various aromatic dibasic acids at a molar ratio of 2:1 is disclosed. The possible problem of gel formation during the reaction was not observed.

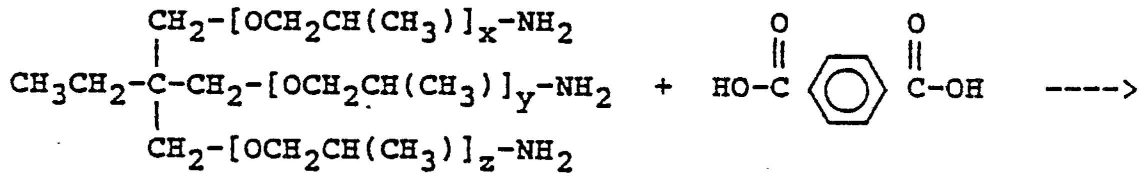

(X)

T-403

Terephthalic Acid

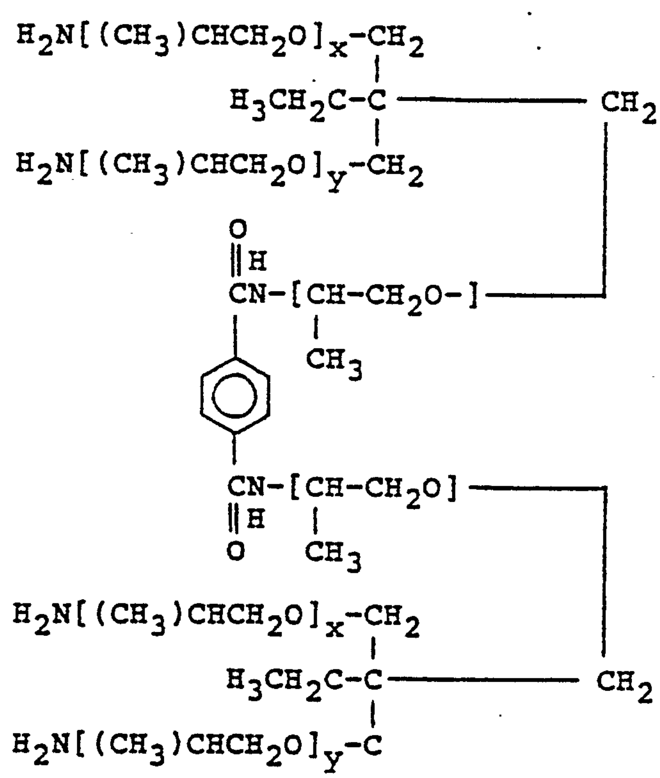

(XI)

<u>Example 2 (6152-14): The Reaction of Jeffamine® T-403 amine and Terephthalic Acid (2:1 Molar Ratio)</u>

To a 500-ml 3-necked flask equipped with a thermometer, mechanical stirrer, Dean-Stark trap and $N_2$-line was charged Jeffamine T-403 (202g ca. 0.5 mole) and terephthalic acid (41.5 g, ca. 0.25 mole). The reaction mixture was heated to 200 °C with stirring and nitrogen flow, for four hour period of time. During this process, 9.0g (0.5 mole) of water was generated and removed through the Dean-Stark trap. After cooling to room temperature, a pale-yellow, viscous product (227.8g) was recovered. The product was insoluble in water, but soluble in methanol and acetone. The total amine assay showed 3.57 meq/g (theoretical 3.7 meq/g based on Jeffamine T-403 at 6.45 meg/g). The acidity titration showed 0.03 meq/g, indicated the high conversion of adipic acid. The IR showed the amide absorption and polyether functionality.

Identical experimental procedures, as described in Example 2 were used, and the results are summarized in the following table:

TABLE II

| PROPERTIES OF PRODUCTS FROM JEFFAMINE T-SERIES AMINES WITH VARIOUS DIBASIC ACIDS AT 2:1 MOLAR RATIO | | |
|---|---|---|
| | T-403 | T-5000 |
| Terephthalic Acid | 6152-14<br>Yellow, viscous transparent liquid<br>Amine 3.71 meq/g<br>Acidity <0.003 meq/g<br>IR : Amide | 6103-83<br>Nearly colorless opaque liquid<br>Amine 0.43 meq/g ,<br>Acidity 0.12 meq/g<br>IR : Amide |
| Isophthalic Acid | 6152-15<br>White, slightly viscous, transparent liquid<br>Amine 3.66 meq/g<br>Acidity 0.32 meq/g<br>IR : Amide | 6152-18<br>Light yellow, transparent liquid<br>Amine 0.39 meq/g<br>Acidity 0.07 meq/g<br>IR : Amide |

Uses of the products of Example 2 include their application in the preparation of polyureas (by reaction with polyisocyanates), epoxy curing agents and crosslinking agents.

Amidoamines from t-Butyl Isophthalic Acid and Polyoxypropylene Diamines and Triamines

Using an Amoco product - t-butyl isophthalic acid and JEFFAMINE® amine manufactured by the Texaco Chemical Company, including JEFFAMINE® EDR-148, EDR-192, D-230, D-2000, ED-2001, T-403, T-3000 and T-5000, a series of amidoamines have been prepared.

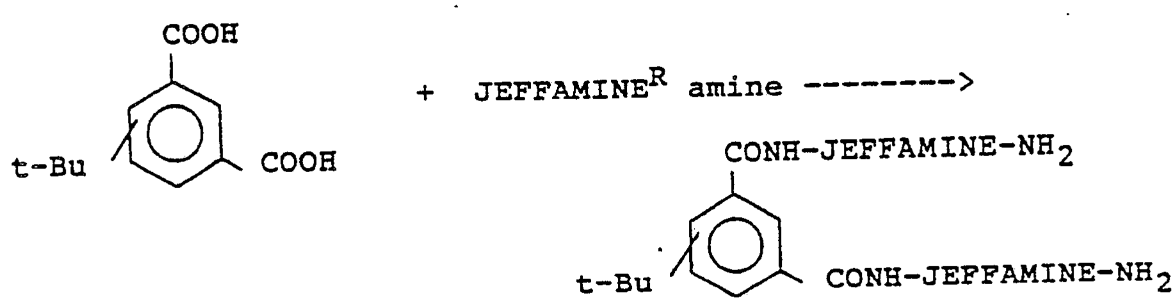


(XII)

These amines can be active amines, (from EDR-amine series), tetrafunctional amine (from T-series amines), water-soluble amine (from Ed-2001), etc. The applications include epoxy curing material, polyamide or polyurea uses.

The alkyl and phenyl groups in the structure can be an important factor contributing to the specific applications, such as increasing compatibility with the monomer component.

Example 3: t-Butyl Isophthalic Acid + EDR-148 (1:2)

To a 500 ml 3-necked flask equipped with a thermometer, Dean-Stark trap, stirrer and $N_2$-line was charged t-butyl isophthalic acid (Amoco t-butyl IPA, 111g, 0.5M) and EDR-148 (Texaco product, 148g, 1.0M). The mixture was heated to 185-215 °C for four hours to remove water (ca. 22 cc). After cooling to room temperature, a yellow solid was obtained (226.5g). The analysis indicated total amine 3.81 meq/g (theoretical 4.1 meq/g) and acidity 0.06 meq/g.

Other examples involving EDR-192, D-230, D-2000, ED-2001, T-403, T-3000 and T-5000 are summarized in the attached table.

TABLE III
AMIDOAMINES FROM T-BUTYL ISOPHTHALIC ACID AND JEFFAMINE$^R$ AMINES

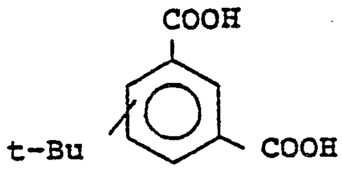

+ JEFFAMINE$^R$ amine -------->

(XII)

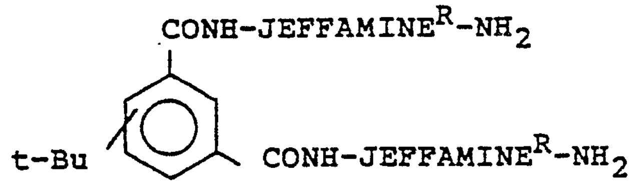

| Notebook No. | JEFFAMINE$^R$ Amine* | Properties of Product |
|---|---|---|
| 6199-57 | EDR-148 | Semisolid yellow<br>Amine 3.81 meq/g (4.1)<br>Acidity 0.06 meq/g |
| 6199-60 | EDR-192 | Solid viscous, transparent, light yellow<br>Amine 3.32 meq/g (3.3)<br>Acidity 0.06 meq/g |
| 6199-61 | D-230 | Solid white transparent<br>Amine 2.41 meq/g (2.9)<br>Acidity 0.15 meq/g |
| 6199-68 | D-2000 | Liquid brown, transparent<br>Amine 0.43 meq/g (0.47)<br>Acidity 0.11 meq/g |
| 6199-72 | ED-2001 | Solid light brown, mp 42, water soluble<br>Amine 0.59 meq/g<br>Acidity 0.10 meq/g |
| 6199-69 | T-403 | Liquid viscous, light yellow, transparent<br>Amine 3.56 meq/g (3.57)<br>Acidity 0.08 meq/g |
| 6199-70 | T-3000 | Liquid light yellow, transparent<br>Amine 0.57 meq/g<br>Acidity 0.08 meq/g |
| 6199-73 | T-5000 | Liquid yellow, transparent<br>Amine 0.33 meq/g (0.39)<br>Acidity 0.69 meq/g |

* Molar ratio of t-BuIPA : Amine = 1:2

Example 3-A: Usage of Product T-BuIPA + T-403 (from 6199-69)

The mixture of 6199-69 product 21.0g and Epon 828 (from Shell 28g) was mixed thoroughly and poured into a mold and cured at 80 °C overnight. A yellowish white rigid material was made.

Amidoamines from Trimesic Acid and Polyoxyethylene or Polyoxypropylene Diamines or Triamines

Using trimesic acid as the coupling agent, a series of new amidoamines can be prepared. The synthesis is described as follows:

(XIII)

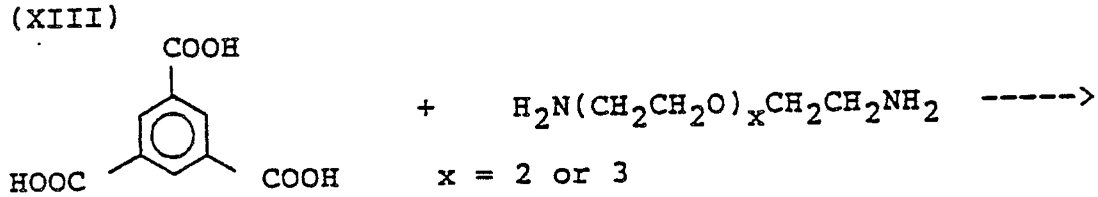


(XIV)

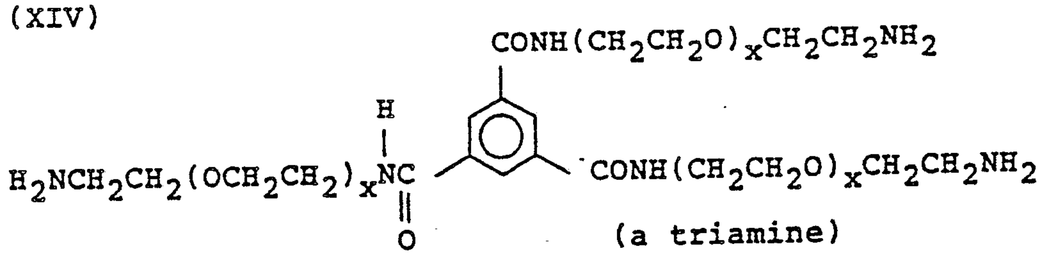


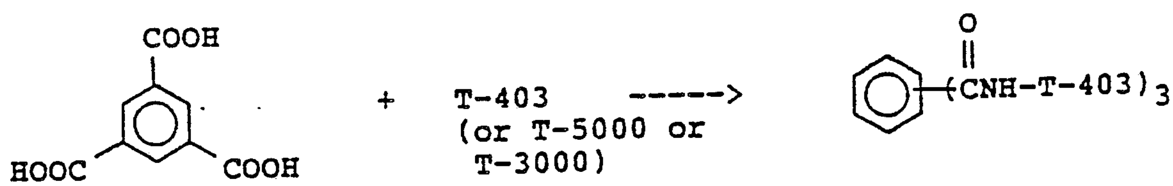

Light colored liquids were obtained from T-series amines and D-2000. Light colored transparent solids were obtained from EDR-series amines. A crystalline yellowish solid was obtained from ED-6000.

Example 4 (6152-25)

To a 250 ml 3-necked flask equipped with a thermometer, Dean-Stark trap, stirrer and $N_2$-line was charged with JEFFAMINE®T-403 (182g, 0.396 mole) and trimesic acid (44.4g, 0.21 mole). The mixture was heated to 200 °C for four hours. during the process, ca. 6.0 cc water was collected in the Dean-Stark trap. After cooling to room temperature, a transparent, light-yellow liquid was recovered (204g) that was non-pourable at room temperature. The analysis showed 3.65 meq/g amine, 0.26 meq/g acid. The IR showed the formation of amide.

Results of other examples, including T-403 at various ratios, T-5000, T-3000, D-2000, D-4000, ED-6000 are in the attached table. The comparative example using citric acid showed the difference between two tribasic acids. The result of trimesic acid to T-403 at 1:1 molar ratio, giving unworkable cross-linkage polyamide indicated the importance of feedstock ratio.

Example 4-A (6152-36)

In a similar manner, three moles of Jeffamine® T-403 amine was allowed to react with one mole of trimesic acid. The resulting product was almost colorless and showed 4.05 meq/g of amine (theory 3.8) and 0.17 meq/g of acid. This product (11.4g) was mixed with 18.5g of Epon 828 (Shell). The reactants began to react very slowly and after two hours the mixture was very thick, but still stirable. The mixture was used to seal two steel coupons together. The reactants cured to an extremely hard resin.

TABLE IV

| PROPERTIES OF PRODUCTS FROM TRIMESIC ACID AND JEFFAMINE AMINES | | | | | | |
|---|---|---|---|---|---|---|
| Notebook No. | Amine | Mole Ratio of Acid to Amine | Properties | ←Analysis→ | | |
| | | | | $-NH_2$, meq/g | -COOH meq/g | IR Amide |
| 6152-25 | T-403 | 1:2 | Transparent, light yellow, viscous liquid-or-solid | 3.65 | 0.26 | Yes |
| 6152-37 | T-403 | 1:1 | Hard solid at 200 °C | - | - | |
| 6152-36 | T-403 | 1:3 | Transparent, light yellow, Fluidable liquid, Sol. in MeOH, Insol. in $H_2O$ | 4.05 | 0.17 | Yes |
| 6152-35 | T-3000 | 1:3 | Nearly colorless clear liquid | 0.60 | 0.06 | Yes |
| 6152-32 | T-5000 | 1:3 | Transparent, light yellow liquid, sol. in MeOH | 0.40 | - | Yes |
| 6152-26 | EDR-192 | 1:3 | Soft, sticky solid (golden yellow) sol. in MeOH, insol. in acetone, $H_2O$ | 3.20 | 0.14 | Yes |
| 6152-39 | EDR-148 | 1:3 | Soft, sticky solid (golden yellow) sol. in MeOH, insol. in acetone, $H_2O$ | 4.90 | 0.11 | |
| 6152-40 | D-4000 | 1:3 | Transparent, light yellow liquid | 0.24 | 0.06 | |
| 6152-42 | ED-6000 | 1:3 | Opaque, light grey crystalline solid | 0.18 | 0.10 | |
| 6152-43 | (with citric acid) | 1:3 | Black, viscous liquid | - | - | |
| 6152-53 | D-2000 | 1:3 | Light yellow liquid, sol. in MeOH, insol. in $H_2O$ | 0.54 | 0.12 | Yes |

Di- and Tetraamidoamines from 2,6-Naphthalene Dicarboxylate and Polyoxyethylene and Polyoxypropylene Diamines and Triamines

A series of novel di- and tetra-amines have been prepared from the reaction of JEFFAMINE® amines and dimethyl 2,6-naphthalene dicarboxylates according to the following reaction:

(XV)

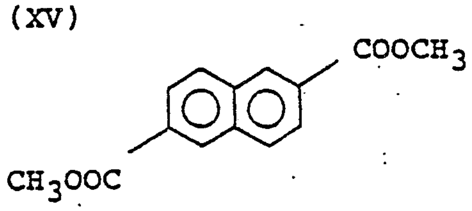

+ JEFFAMINE$^R$ amine ----->

(XVI)

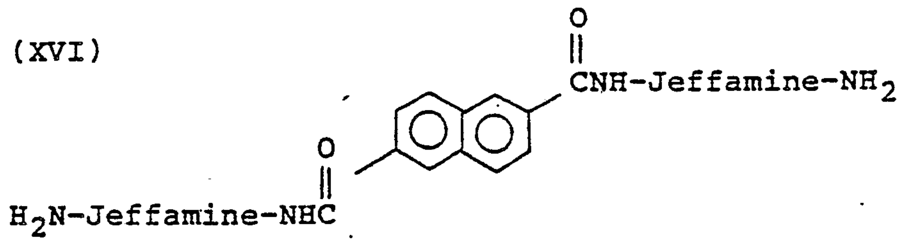

(where JEFFAMINE® amine = EDR-192, D-2000, D-4000, ED-6000, ED-2001, T-403 or T-5000).

These amines, containing a naphthalene-functionality can be a diamine (from JEFFAMINE® diamine), a tetra-amine (from JEFFAMINE triamine) or a water soluble amine (from JEFFAMINE ED-series amine). In comparative examples, in contrast to dimethyl 2,6-naphthalene dicarboxylate another precursor, 2,6-naphthalene dicarboxylic acid afforded decomposing products. These results are summarized in a table. One of the uses of these products as an epoxy resin was demonstrated.

## Example 5: Reaction of DM-2,6-NDC and JEFFAMINE® T-403 (1:2 Molar Ratio) (6153-4)

To a 250-ml 3-necked flask equipped with thermometer, mechanical stirrer, Dean-Stark trap and $N_2$-inlet-outlet line, charge dimethyl, 2,6-naphthalene dicarboxylate (Amoco product 36.6g, 0.15M) and JEFFAMINE® T-403 (138g, 0.3 mole). The mixture was heated to 200 °C, under nitrogen atmosphere and held for 2.5 hours. After cooling to room temperature, a highly viscous, light colored liquid was obtained (157g). The analysis indicated amine content 3.79 meq/g, acidity 0.29 meq/g. IR spectrum showed the formation of amide.

## Example 5-A: Usage of DM-2,6-NDC-T-403 Adduct (6153-4A)

A mixture of product (from 6153-4) (39.6g, ca. 0.15 meq) and Epon 828 (Shell product) (56.1g, 0.30 meq/g) was stirred thoroughly and then poured to a mold. The mixture was cured at ca. 70 °C. A rigid epoxy resin material was made.

Other examples are cited in Table V.

## Example 5-B: Comparative Example Using 2,6-Naphthalene Dicarboxylic Acid (6152-73)

Following Example 5 procedures, a mixture of 2,6-naphthalene dicarboxylic acid (10.8g) and JEFFAMINE D-2000 (200g) was heated to 200-220 °C for a few hours. The final product obtained was dark blue liquid with analysis of 0.84 meq/g amine and 0.42 meq/g acidity. The use of 2,6-naphthalene dicarboxylic acid gave a decomposed product. These negative results indicated the importance of using a suitable precursor - diester of 2,6-naphthalene dicarboxylic acid.

## TABLE V

## DI- AND TETRA-AMINES FROM DIMETHYL-2,6-NAPHTHALENE DICARBOXYLATE AND JEFFAMINE[R] AMINES

(XVII)

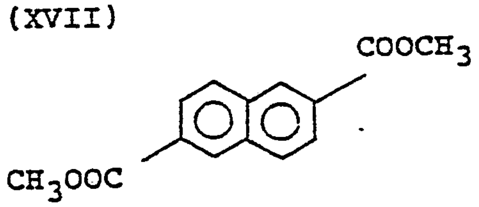

+ JEFFAMINE[R] amine ----->

(XVIII)

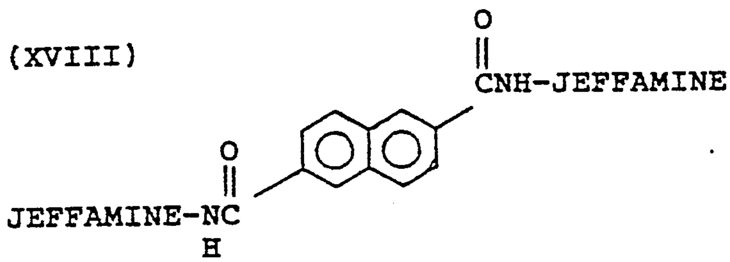

| Notebook No. | JEFFAMINE[R] Amine | Products: Analysis* -NH2 | -COOH | Properties |
|---|---|---|---|---|
| 6152-99 | EDR-192 | 3.14<br>3.3 | 0.26 | Solid white<br>mp ca. 75°C<br>IR : Amide |
| 6153-2 | EDR-148 | - | - | mp > 200°C |
| 6153-35 | D-2000 | 0.66<br>(0.47) | 0.16 | Liquid brown<br>IR : Amide |
| 6153-14 | D-400 | 1.98<br>(1.90) | 0.41 | Solid waxy yellow<br>IR : Amide |
| 6153-13 | ED-600 | 1.60<br>(1.3) | 0.43 | Liquid trans-parent yellow<br>Water soluble |
| 6153-97 | ED-2001 | 0.65<br>(0.48) | 0.14 | Solid crystalline<br>Water soluble |
| 6153-4 | T-403 | 3.79<br>(3.50) | 0.29 | Liquid yellow, viscous |
| 6153-81 | T-5000 | 0.44<br>(0.4) | 0.06 | Liquid yellow |

*Analysis: Amine and acidity in meq/g. Calculated figures in parenthesis.

Amidoamines from Nonconjugated Aromatic Dicarboxylic Acids and Polyoxypropylene Diamines and Triamines and Polyoxyethylene Diamines

AMOCO® PIDA (1,1,3-trimethyl-3-phenylindan-4′,5-dicarboxylic acid) is a "non-conjugated", non-planar diacid, containing both alkyl- and aromatic functionalities. The uses of this diacid enable us to prepare a series of novel amines, from the reactions of PIDA and JEFFAMINE® amines including JEFFAMINE T-403, T-3000, T-5000, D-2000, D-4000, ED-600, EDR-192, EDR-148 and BAEE (bis-aminoethyl ether).

(XIX)

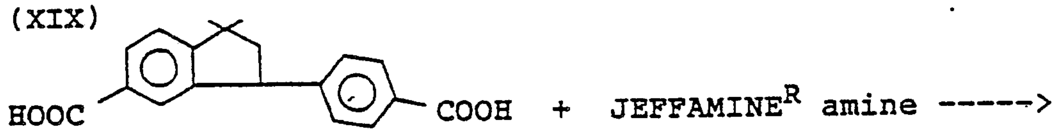


(XX)

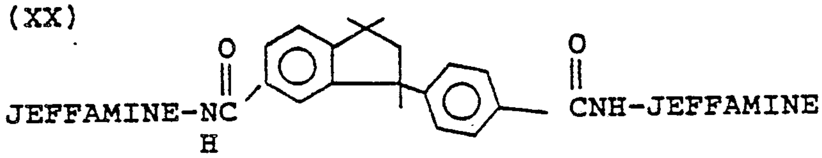

These products are light colored semisolid liquids and soluble in alcohol solvents, except (1) the product from ED600 was soluble in water with the phenomenon of inverse solubility factor at 29-41 °C and (2) the product from BAEE could not be prepared under the reaction conditions we used, due to the high melting point of this amide-acid salt.

The product will be useful in a number of applications, such as polyurea and epoxy resin. A specific usage in the area of epoxy resin has been exemplified.

Example 6 (6152-48)

To a 250-ml 3-necked flask equipped with thermometer, stirrer, Dean-Stark trap and $N_2$ inlet-outlet line was charged AMOCO® PIDA (12.1g, 0.037 mole) and JEFFAMINE® D-2000 amine (150g, 0.075 mole). The mixture was heated to 210 °C for 2 hours, then cooled to room temperature. A transparent, light brown liquid product was recovered (228g). The analysis showed 0.52 meq/g amine (calc. 0.47 meq/g), acidity 0.15 meq/g and water content 0.06%. The IR showed the formation of amide.

Example 6-A (6152-49)

To a 250-ml 3-necked flask equipped with thermometer, stirrer, Dean-Stark trap and $N_2$ inlet-outlet line was charged AMOCO® PIDA (64.9g, 0.20 mole) and JEFFAMINE® EDR-192 amine (76.8g, 0.40 mole). The mixture was heated to 200 °C for four hours. during the process, ca. 7.0 cc of water was removed through the Dean-Stark trap. After cooling to room temperature, a yellowish semisolid product was recovered (61.5g) This product was soluble in methanol and insoluble in water. The analyses indicated amine, 3.0 meq/g (calc. 3.0 meq/g), acidity 0.08 meq/g. The IR analysis indicated the formation of amide.

Other examples are cited in Table VI.

Example 6-B (6152-48B)

A soap dish was made by mixing 77g of 6152-48 (reaction product from AMOCO® PIDA and JEFFAMINE® D-2000) and 15 g of Epon 828 (diglycidol ether of bisphenol-A from Shell) and pouring the contents in an elastic mold. The material was cured overnight in an oven set at 80 °C. The material was soft and rubbery solid.

Example 6-C (6152-48B)

Repeated the previous example using the mixture of 48g of 6152-48, 6.6g of dipropyl-triamine (DPTA) and Epon 828 56.1g. After curing at 80 °C for overnight, a hard solid with some flexibility was made.

TABLE VI

| PROPERTIES OF PRODUCTS FROM AMOCO PIDA AND JEFFAMINE® AMINES | | | | | |
|---|---|---|---|---|---|
| Notebook No. | Amine | Molar Ratio of Acid to Amine | Analyses | | Notes |
| | | | $-NH_2$ meq/g | -COOH meq/g | |
| 6152-68 | T-403 | 1:2 | 3.4 | 0.21 | Yellow, semisolid |
| 6152-66 | T-3000 | 1:2 | 0.63 | 0.08 | Light color liq. |
| 6152-89 | T-5000 | 1:2 | 0.38 | 0.11 | Yellow liquid |
| 6152-71 | T-5000 | 1:3 | 0.40 | 0.08 | Yellow liquid |
| 6152-48 | D-2000 | 1:2 | 0.52 | 0.15 | Transparent, light-brown liq. |
| 6152-65 | D-4000 | 1:2 | 0.26 | 0.10 | Brown liquid |
| 6152-69 | ED-600 | 1:2 | 1.26 | 0.13 | Light yellow liq. soluble in $H_2O$ ISF at 29-41 °C |
| 6152-49 | EDR-192 | 1:2 | 3.0 | 0.08 | Yellow, semisolid |
| 6152-50 | EDR-148 | 1:2 | 4.2 | 0.25 | Yellow solid |
| 6152-51 | BAEE | 1:2 | - | - | High m.p. salt |
| *Products are soluble in MeOH and insoluble in $H_2O$, except ED-600 product. | | | | | |

The foregoing examples have been given by way of illustration only and are not intended as limitations on the scope of this invention, which is defined by the appended claims.

Detailed Description of another Embodiment

The amidoamine reaction products used as curing agents of uncured epoxy resins in the present invention are reaction products which contain as the principle reaction component, an amidoamine having either formula I:

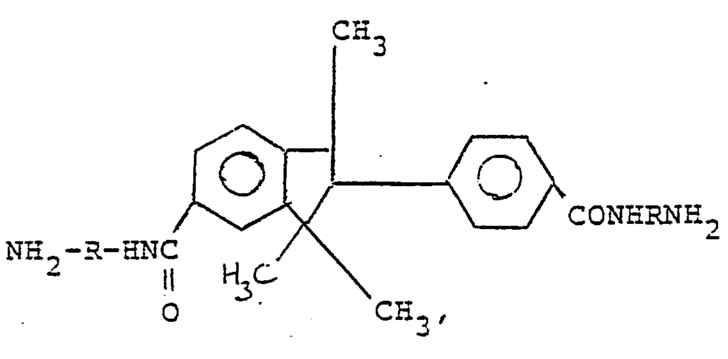

(I)

which is the reaction product of phenylindane dicarboxylic acid, (1,1,3-trimethyl-3-phenylindane-4′,5-dicarboxylic acid) and a polyether amine wherein R is a polyether segment, either polyoxypropylene or polyoxyethylene or both in combination, or formula II:

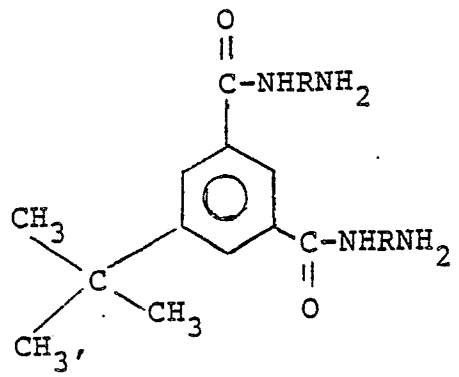

(II)

which is the reaction product of t-butyl isophthalic acid and a polyether amine, wherein R has the same meaning as in (I) above.

Encompassed within the above formulas are reaction products of dicarboxylic acids with polyoxyalkylene diamines which will contain two terminal primary amine groups.

In the instant invention it has been discovered that these amidoamine reaction products of dicarboxylic acids and polyether amines have improved properties which make them valuable as epoxy curing agents. When combined with an epoxy resin in proper stoichiometric amounts these amidoamines result in a rapid curing system, even under ambient conditions. Excellent properties are obtained on curing of these epoxy amidoamine systems. Such combinations are useful in a number of applications such as coatings, adhesives, encapsulations, laminates, composite fabrications and sealants.

Composition of the amidoamine is apparently critical in obtaining the improved curing properties. The polyetherpolyamine portion may be polyoxypropylene or polyoxyethylene or combinations of these and may range in molecular weight from ~200 to ~6000. The composition of the dicarboxylic acid is critical with regard to increased compatibility with epoxy resin, and thus leads to enhanced properties over similar amidoamines.

Preferred dicarboxylic acids are aromatic.

The dicarboxylic acid used as a reactant for the present invention may be any suitable aromatic carboxylic acid having functionality of ≥2. The anhydrides and lower alkyl esters of the acids wherein the alkyl group contains 1 to 4 carbon atoms, and is preferably methyl, may also be used. Examples of aromatic dicarboxylic acids that may be used as starting materials for the present invention include acids such as terephthalic acid, isophthalic acid, trimesic acid, 1,1,3-trimethyl-3-phenylindane-4′,5-dicarboxylic acid, 2,6-naphthalene dicarboxylic acid, t-butyl isophthalic acid, etc. Also included are benzene dicarboxylic acids, hemimellitic acid, 2-phenyl pentanedioic acid, phenyl succinic acid, trimellitic, etc.

The preferred dicarboxylic acids are phenylindane (1,1,3-trimethyl-3-phenylidane-4′,5-dicarboxylic acid) and t-butyl isophthalic acid.

The polyetherpolyamine starting materials for the present invention may include several different amines. In general, the polyetherpolyamine starting material may be defined as a polyetherpolyamine having the formulas:

1.

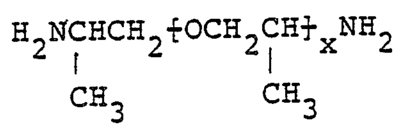

where x is from 2.6 to 33.1 ;

2. $H_2NCH_2CH_2(OCH_2CH_2)_x$-$NH_2$ ;

x = 0,1,2

3. $H_2NCH(CH_3)CH_2$ $OCH(CH_3)(CH_2$ $_x$ $OCH_2CH_2$ $_y$ $OCH_2CH(CH_3)$ $_zNH_2$ ; Amines with functionality of >2 of the following formula cannot be used in preparation of amidoamines.

4.

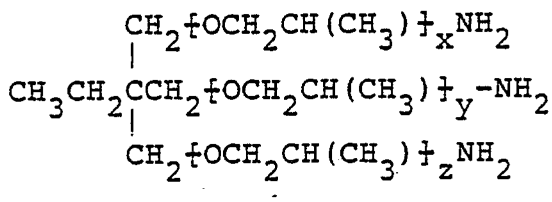

In general, the average molecular weight of the polyoxypropylene diamine starting material will be from about 200 to about 6000.

An advantage is obtained when using lower molecular weight diamine starting materials such as those having average molecular weights of about 200 to about 3000 in that the final products will have primary amine functionalities and higher molecular weights but will not contain contaminants of the type formed by the oxypropylation of dihydric and trihydric alcohols with propylene oxide to form polyoxypropylene diols and triols having molecular weights of 3000 to 5000.

One group of appropriate polyoxyalkylene diamines, containing both ethylene oxide and propylene oxide, which may be used as polyoxypropylene diamines having the formula:

(IV) 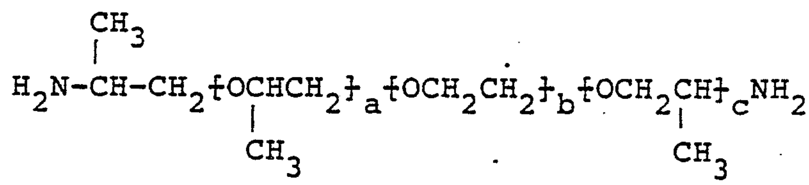

wherein a + c equals a number having a value of from about 2 to about 3 and b is a number having a value of from about 5 to about 45. The preferred amines include polyoxypropylene and polyoxyethylene or a combination thereof.

It will be apparent to those skilled in the art that many of the suitable polyoxyalkalene polyamines may be available as and used as commercial mixtures of several components. Useful commercial materials include those available under the trademark JEFFAMINE® D-, T- and ED-, etc., marketed by Texaco Chemical Co.

Commercial products used in the examples of this invention include JEFFAMINE® EDR-148 and JEFFAMINE® EDR-192. These are polyoxyalkylene amines produced by Texaco Chemical Co. under the name JEFFAMINE® ED Series Amines.

It has been discovered that an amidoamine addition product is preferentially formed when an aromatic dicarboxylic acid is reacted with an excess of a polyoxyalkylene diamine at autogenous pressure at a temperature within the range of about 150° to about 250° C for a reaction time within the range of about 0.5 to about 12 hours.

The reaction to form the amidoamines may be carried out in the absence of added solvent-diluent, but the latter may be present if desired including water, toluene, etc. By-product water of reaction is preferably removed from the reaction mixture as formed. The reaction is complete when essentially all of the carboxylic groups have reaction with primary amine groups of the polyoxyalkylene diamine or triamine. Under the noncatalytic reaction conditions employed herein, the primary amine groups of the polyoxyalkylene diamine or triamine are essentially unreactive with each other.

The amidoamines that are formed are liquid or amorphous solid materials having a molecular weight within the range of about 400 to about 6000 and containing usually 2 terminally primary amine groups.

It can be demonstrated that the novel amidoamines described above and in copending U.S. Application Serial No. (Attorney's Docket #80,616), when combined with an epoxy resin, provide improved curing properties.

An epoxy resin which may be cured by the process of this invention may typically be one prepared for example by the reaction of Bisphenol A with epichlorohydrin in the presence of sodium hydroxide. After condensation is complete, the crude resin is freed of residual epichlorhydrin, washed well to remove salt and soluble by-products, and recovered.

Curing of such a resin is effected in the presence of variable amounts of the amidoamine of this invention. Preferably the epoxy resin is a liquid. Among those which have demonstrated the effectiveness of the instant invention are diglycidyl ethers of Bisphenol A, such as liquid epoxy resin EEW~185. (EEW = epoxy equivalent weight; molecular weight of resin = ~380; functionality ~ 2; equivalent weight ~ 185-190.)

The novel amidoamine should be present in the epoxy resin in an amount sufficient to provide about 0.8 to 1.2 amino ($NH_2$) groups per oxirane group of the epoxy resin.

Practice of the novel method of this invention may be apparent from the following description in Examples I through VII of the preferred embodiment.

This method of curing epoxy resins with the amidoamines herein described allows for the production of compositions with properties which would have a number of useful applications such as coatings, adhesives, incapsulations, laminates, composite fabrications and sealants. Many properties which can be obtained by variations of the invention are demonstrated in Examples I through VII.

Thus, Examples I and III through VII demonstrate the method of this invention for curing epoxy resins with novel amidoamines to provide compositions with excellent coating properties.

In the Examples which represent the process of this invention, the preparation of a cured epoxy resin is carried out in the following manner:

Epoxy resin is normally used without dilution and without other additives. The curing agent(s) in this case were the more viscous component(s). Solvents (butanol), therefore, were added to the curative component along with leveling agent.

A component is prepared containing variable amounts, of liquid epoxy resin, butanol and leveling agent.

To this compound is added an equivalent amount of the amidoamine. The mixture is then mixed, degassed (Degassed-freed of entrapped air through application of vacuum). and poured into molds. After curing, the cured materials are cut into test samples by precision methods.

In the various Examples, the following properties are measured.

Drying Time - a reproducible determination of various stages and rates of film formation in drying and curing of organic coatings Gardner Circular Drying Time Recorder was used. Pencil Hardness - film hardness rated according to hardness of lead pencil [4B(soft) to 6H (hard)] required to scratch or gauge a film ASTM D3363-74. Impact - as measured by Gardner impact, in-lbs. to fail. A known weight is allowed to fall from a measured height to exert a certain force upon a film sample by means of a round-nose punch. After striking, the film is examined for damage. ASTM D2794-69 (1974). Tensile Shear Strength - ASTM D 1002 - Strength properties, in psi, of an adhesive when loaded to produce shear distortion of planes parallel to the plane of the adhesive bond. T-peel strength, pli - ASTM D 1876 - a test made on bonded strips of metals by peeling the metal strips back and recording the adhesive strength values.

The amidoamine of Example I is that formed by condensing phenylindase dicarboxylic acid with a polyetheramine. The resin used is EEW~185 Epon 828; liquid epoxy resin of equivalent weight ~185-190.

In the examples demonstrating compositions with coating properties a leveling agent is used in order to aid in the uniform dispersion of the components of the curing system. The properties observed in the product indicate suitability as a coating. Those of ordinary skill in the art may see other applications.

Examples III demonstrates the superiority of properties obtained on curing an epoxy resin with an amidoamine prepared from t-butyl isophthalic acid rather than isophthalic or dimer acid.

The data of Example IV shows that the condensation product amidoamine of t-BuIPA and EDR-192 cures more slowly than amidoamines prepared from EDR-148, however the coatings exhibited more flexibility in addition to exhibiting more toughness and hardness.

Additionally, it can be seen in Examples V and VI that the addition of JEFFAMINE® 230 with amidoamine condensation products previously demonstrated allows for even more rapid curing and hardening of the surface without compromising the desirable properties demonstrated in Examples I and IV.

Example II demonstrates the desirable adhesive properties obtained by using an amidoamine produced from phenylindane dicarboxylic acid.

Example 1 :

| Coatings Properties : Epoxy System Cured with Amidoamines from Phenylindane Dicarboxylic Acid (PIDA) and Polyetheramines. | | |
|---|---|---|
| Formulation | A | B |
| Epoxy resin (EEW~185) | 100 | 100 |
| PIDA JEFFAMINE® EDR=148 amidoamine | 64 | - |
| PIDA JEFFAMINE® EDR-192[2] amidoamine | - | 78 |
| n-Butanol | 13.1 | 14.2 |
| Leveling agent[3] | 3.3 | 3.6 |
| Coating Properties: | | |
| Drying time, 6-mil film | | |
| Set to touch, hrs. | 1.2 | 3.5 |
| Surface-dry, hrs. | 4.3 | 6.5 |
| Thru-dry, hrs. | - | 10.4 |
| Pencil hardness after 24 hrs.,~25° C | HB-F | B |
| 7 days,~25° C | B-HB | B |
| 24 hours, 25° C, 1 hrs., 125° C | H | H |
| Gardner impact, in-lbs to fail (dir./rev.) after 24 hours,~25° C | 16/<4 | >160/>160 |
| 7 days,~25° C | 20/<4 | >160/>160 |
| 24 hrs., 25° C, 1 hr., 125° C | 28/<4 | 40/8 |
| 1 Structure = $H_2NCH_2CH_2OCH_2CH_2OCH_2CH_2NH_2$ | | |

2 Structure = $H_2NCH_2CH_2OCH_2CH_2OCH_2CH_2OCH_2CH_2NH_2$

3 Beetle 216-8 (American Cyanamid Co.)

Example 2 :

| Adhesive Properties : Epoxy System Cured with Amidoamines from Phenylindane Dicarboxylic Acid (PIDA) and Polyether Amines | | |
|---|---|---|
| Formulation | A | B |
| Liquid epoxy resin (EEW~185) | 100 | 100 |
| PIDA JEFFAMINE® EDR-148 Amidoamine | | |
| PIDA JEFFAMINE® EDR-192 Amidoamine | | |
| Adhesive Properties:[1] | | |
| Tensile Shear Strength, psi | 3900 | 5000 |
| T-peel strength, pli | 3.2 | 6.8 |

[1]Cure cycle : overnight at 25° C, one hour, 125° C.

Example 3 : Coatings Properties : Epoxy Resin Cured with Amidoamine Prepared from T-Butyl Isophthalic Acid (T-BuIPA), Other Dibasic Acids and JEFFAMINE® EDR-148[5]

| Formulation | A | B | C |
|---|---|---|---|
| Liquid Epoxy resin (EEW~185) | 100 | 100 | 100 |
| t-BuIPA JEFFAMINE® EDR-148[5]) Amidoamine | 76 | - | - |
| Isophthalic acid JEFFAMINE® EDR-148 Amidoamine - | 58 | - | |
| Dimer Acid JEFFAMINE® EDR-148 Amidoamine | - | - | 125 |
| n-Butanol | 14.5 | 12.6 | 11.25 |
| Leveling agent[1] | 3.5 | 3.2 | 4.5 |
| **Coatings Properties** | | | |
| Drying time, 6-mil film | | | |
| Set-to-touch, hours | 2.5 | 1.6 | 5.1 |
| Surface-dry, hours | 6.2 | 3.7 | 6.3 |
| Thru-dry, hours | 7.2 | >24 | >30 |
| Pencil hardness | | | |
| After 24 hours, ~25°C | 2B | >3B[2] | 3B[3] |
| After 72 hours, ~25°C | HB | - | - |
| After 7 days, ~25°C | F | >3B[2] | >3B[3] |
| 24 hrs, 25°C, 1 hr. 125°C | 2H | 3B-2B | >3B[4] |
| Gardner impact, in-lbs to fail: (dir/rev) | | | |
| After 24 hours, ~25°C | 40/8 | 160/140 | >160/>160 |
| After 72 hours, ~25°C | 32/<4 | - | >160/>160 |
| After 7 days, ~25°C | 12/<4 | 60/~20 | >160/>160 |
| 24 hrs, 25°C, 1 hr. 125°C | 28/<4 | <4/40 | >160/>160 |

[1]Beetle Resin 216-8; American Cyanamide Co.
[2]Soft, tacky coating; undercured.
[3]Considerable oily material formed on surface; apparently incompatible.
[4]Slightly tacky surface;
[5]Structure : $H_2NCH_2CH_2OCH_2CH_2OCH_2CH_2NH_2$

Comment: Amidoamine prepared from t-BuIPA and EDR-148 resulted in cured coatings with much better properties than with those prepared with EDR-148 and either isophthalic acid or dimer acid. The amidoamine prepared with t-BuIPA cured more rapidly and formed coatings that were harder with a more durable surface.

Example 4 : Coatings Properties : Epoxy Resin Cured with Amidoamine Prepared from T-BuIPA or Other Dibasic Acids and JEFFAMINE® EDR-192[1]

| Formulation | A | B | C |
|---|---|---|---|
| Liquid Epoxy resin (EEW~185) | 100 | 100 | 100 |
| t-BuIPA JEFFAMINE® EDR-192) Amidoamine | 80 | - | - |
| Isophthalic acid JEFFAMINE® EDR-192 Amidoamine - | 70 | - | |
| Dimer Acid JEFFAMINE® EDR-192[1] Amidoamine | - | - | 115 |
| n-Butanol | 14.4 | 13.6 | 10.75 |
| Leveling agent[2] | 3.6 | 3.4 | 4.3 |
| **Coatings Properties** | | | |
| Drying time, 6-mil film | | | |
| Set-to-touch, hours | 5.0 | 3.2 | 6.2 |
| Surface-dry, hours | 7.2 | 5.0 | 8.3 |
| Thru-dry, hours | 20.4 | 23.6 | 11.3 |
| Pencil hardness | | | |
| After 24 hours, ~25°C | 2B | >3B[3] | 3B[4] |
| After 72 hours, ~25°C | F | - | 3B |
| After 7 days, ~25°C | HB | >3B[3] | >3B[3] |
| 24 hrs, 25°C, 1 hr. 125°C | H | HB | HB |
| Gardner impact, in-lbs to fail: (dir/rev) | | | |
| After 24 hours, ~25°C | >160/>160 | >160/>160 | >160/>160~ |
| After 72 hours, ~25°C | >160/>160 | - | >160/>160 |
| After 7 days, ~25°C | >160/>156 | >160/>160 | >160/>160 |
| 24 hrs, 25°C, 1 hr. 125°C | 32/60 | 12/40 | >160/>160 |

[1] Structure : $H_2NCH_2CH_2OCH_2CH_2OCH_2CH_2OCH_2CH_2NH_2$
[2] Bettle Resin 216-8; American Cyanamide Co.
[3] Soft, tacky, undercured coating
[4] Slightly tacky surface
[5] Blushed surface

Comment: Coatings cured with t-BuIPA EDR-192 amidoamine were of better quality than were those cured with other amidoamines. Although curing was slower than with the amidoamine prepared from EDR-148, coatings were more flexible but were also tough and hard.

Example 5 :

| Coatings Properties : Cures with JEFFAMINE® D-230, Blends of D-230 with t-BuIPA EDR-148 Amidoamine | | | |
|---|---|---|---|
| Formulation | A | B | C |
| Liquid Epoxy resin (EEW~185) | 100 | 100 | 100 |
| JEFFAMINE® D-230/t-BuIPA EDR-148 Amidoamine Blend | | | |
| 100/0 pbw blend | 32 | - | - |
| 80/20 pbw blend | - | 35.5 | - |
| 60/40 pbw blend | - | - | 41.0 |
| n-Butanol | 10.6 | 10.8 | 11.3 |
| Leveling agent[1] | 2.6 | 2.7 | 2.8 |
| Coatings Properties | | | |
| Drying time, 6-mil film | | | |
| Set-to-touch, hours | 7.9 | 5.8 | 4.9 |
| Surface-dry, hours | 17.2 | 9.9 | 7.8 |
| Thru-dry, hours | 23.5 | 16.2 | 18.6 |
| Pencil hardness | | | |
| After 24 hours, ~25° C | B | B | B |
| After 48 hours, ~25° C | B | HB-F | B |
| After 72 hours, ~25° C | HB | F | HB |
| After 7 days, ~25° C | H | H | HB |
| 24 hrs 25° C, 1 hr. 80° C, 1 hr. 125° C | H[2] | H | H |
| Gardner impact, in-lbs to fail: (dir/rev) | | | |
| After 24 hours, ~25° C | 12/<4 | 12/<4 | 16/<4 |
| After 48 hours, ~25° C | 12/<4 | 12/<4 | 16/<4 |
| After 72 hours, ~25° C | 16/<4 | 16/<4 | 16/<4 |
| After 7 days, ~25° C | 16/<4 | 12/<4 | 16/<4 |
| 24 hrs 25° C, 1 hr 80° C, 1 hr 125° C | >160/48[2] | ~80/20 | 60/20 |

[1]Beetle Resin 216-8; American Cyanamide Co.
[2]Cure cycle : 24 hours ~25° C, 1 hr., 125° C
Comments: Blends of JEFFEAMINE® D-230 with t=BuIPA EDR-148 amidoamine result in epoxy coatings that cure more rapidly and surface harden more rapidly than when D-230 is used as a sole curative.

Example 6 :

| Coatings Properties : Cures with JEFFAMINE® D-230 and Blends of D-230 with t-BuIPA EDR-192 Amidoamine | | | |
|---|---|---|---|
| Formulation | A | B | C |
| Liquid Epoxy resin (EEW~185) | 100 | 100 | 100 |
| JEFFAMINE D-230/t-BuIPA EDR-192 Amidoamine Blend | | | |
| 100/0 pbw blend | 32 | - | - |
| 80/20 pbw blend | - | 35.7 | - |
| 60/40 pbw blend | - | - | 41.4 |
| n-Butanol | 10.6 | 10.8 | 11.3 |
| Leveling agent[1] | 2.6 | 2.7 | 2.8 |
| Coatings Properties | | | |
| Drying time, 6-mil film | | | |
| Set-to-touch, hours | 7.9 | 7.4 | 6.8 |
| Surface-dry, hours | 17.2 | 12.8 | 10.8 |
| Thru-dry, hours | 23.5 | - | 17.2 |
| Pencil hardness | | | |
| After 24 hours, ~25° C | B | 3B | 3B |
| After 48 hours, ~25° C | B | H | H |
| After 72 hours, ~25° C | HB | H | H |
| After 7 days, ~25° C | H | H | H |
| 24 hrs 25° C, 1 hr 80° C, 1 hr 125° C | H[2] | H | H |
| Gardner impact, in-lbs to fail: (dir/rev) | | | |
| After 24 hours, ~25° C | 12/<4 | 24/<4 | 20/<4 |
| After 48 hours, ~25° C | 12/<4 | 20/<4 | 28/12 |
| After 72 hours, ~25° C | 16/<4 | 12/<4 | 16/<4 |
| After 7 days, ~25° C | 16/<4 | 20/<4 | 32/8 |
| 24 hrs~25° C, 1 hr 80° C, 1 hr 125° C | >160/48[2] | 160/76 | 160/60 |

[1]Beetle Resin 216-8; American Cyanamide Co.
[2]Cured 24 hours ~ 25° C, 1 hr, 125° C.
Comments: Again, more rapid curing was achieved with blends containing t-BuIPA EDR-192 amidoamine. Coatings cured with blends rapidly achieved a high degree of surface hardness, flexibility.

Example 7 :

| Coatings Properties : Epoxy Resin Cured with JEFFAMINE® D-230, Blends of D-230 with Amidoamine Prepared from t-BuIPA D-230 | | | |
|---|---|---|---|
| Formulation | A | B | C |
| Liquid Epoxy resin (EEW~185) | 100 | 100 | 100 |
| JEFFAMINE D-230/t-BuIPA EDR-230 Amidoamine Blend | | | |
| 100/0 pbw blend | 32 | - | - |
| 80/20 pbw blend | - | 35.3 | - |
| 60/40 pbw blend | - | - | 40.5 |
| n-Butanol | 10.6 | 10.8 | 11.2 |
| Leveling agent[1] | 2.6 | 2.7 | 2.8 |
| Coatings Properties | | | |
| Drying time, 6-mil film | | | |
| Set-to-touch, hours | 7.9 | 7.0 | 6.8 |
| Surface-dry, hours | 17.2 | 13.3 | 13.4 |
| Thru-dry, hours | 23.5 | - | 17.5 |
| Pencil hardness | | | |
| After 24 hours, ~25° C | B | F | F |
| After 48 hours, ~25° C | B | H | F-H |
| After 72 hours, ~25° C | HB | H | H |
| After 7 days, ~25° C | H | H | H |
| 24 hrs 25° C, 1 hr 80° C, 1 hr 125° C | H[2] | H | H |
| Gardner impact, in-lbs to fail: (dir/rev) | | | |
| After 24 hours, ~25° C | 12/<4 | 24/<4 | 16/<4 |
| After 48 hours, ~25° C | 12/<4 | 16/<4 | 16/12 |
| After 72 hours, ~25° C | 16/<4 | 20/<4 | 12/<4 |
| After 7 days, ~25° C | 16/<4 | 16/<4 | 16/<4 |
| 24 hrs~25° C, 1 hr 80° C, 1 hr 125° C | >160/48[2] | >160/160 | >160/>160 |

[1]Beetle Resin 216-8; American Cyanamide Co.
[2]Cure cycle : 24 hours~25° C, 1 hr. 125° C.

## Claims

1. An amidoamine reaction product characterised in that it is formed by reaction or an aromatic di- or tricarboxylic acid, ester of anhydride with a polyetheramine.

2. A reaction product according to claim 1 characterised in that it contains an average of 2-6 terminal primary amine groups and has an average molecular weight of about 400 to 15,000,
said amidoamine having been prepared by reacting an aromatic di- or tricarboxylic acid, ester or anhydride thereof with at least 2 mole equivalents of a polyoxyalkylene polyamine,
the principal amidoamine reaction product being an amidoamine formed by coupling each carboxyl group of said carboxylic acid, ester or anhydride thereof through an amide group with a terminal primary amine group of said polyamine,
said polyoxyalkylene polyamine having the formula:

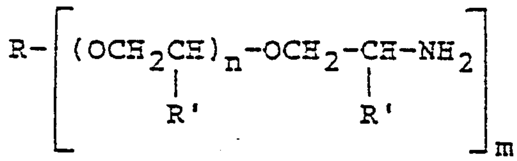

Wherein:
R is the nucleus of an oxyalkylation susceptible polyhydric alcohol containing 2-12 carbon atoms and 2-3 hydroxyl groups, and
R′ is hydrogen or methyl
n is a number having an average value of 0 to 50, and
m is an integer having a value of 2-3.

3. A reaction product according to claim 2 characterised in that it contains two primary amine groups, wherein the aromatic carboxylic acid originally contains two carboxylic acid groups and the polyoxyalkylene polyamine is a polyoxyalkylene diamine.

4. A reaction product according to claim 3 characterised in that the polyoxyalkylene diamine has the formula:

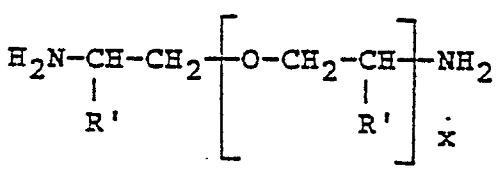

wherein R′ independently represents hydrogen or methyl and x is a number having an average value of about 1 to about 40.

5. A reaction product according to claim 3 characterised in that the polyoxyalkylene diamine has the formula:

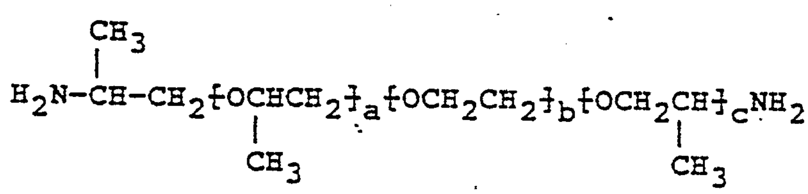

wherein a+c equals a number having a value of about 2 to about 10 and b is a number having a value of from about 1 to about 50.

6. A reaction product according to the preceding claims characterised in that the amine has one of the following formula:

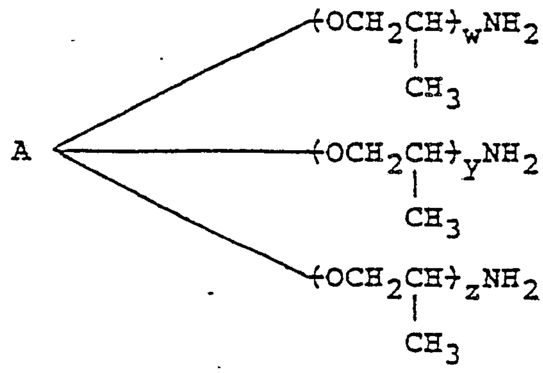

(V)

wherein A represents the nucleus of an oxyalkylation susceptible trihydric alcohol containing from about 3 to about 6 carbon atoms, w, y and z are numbers and the average value of the sum of w + y + z is from about 4 to about 100,

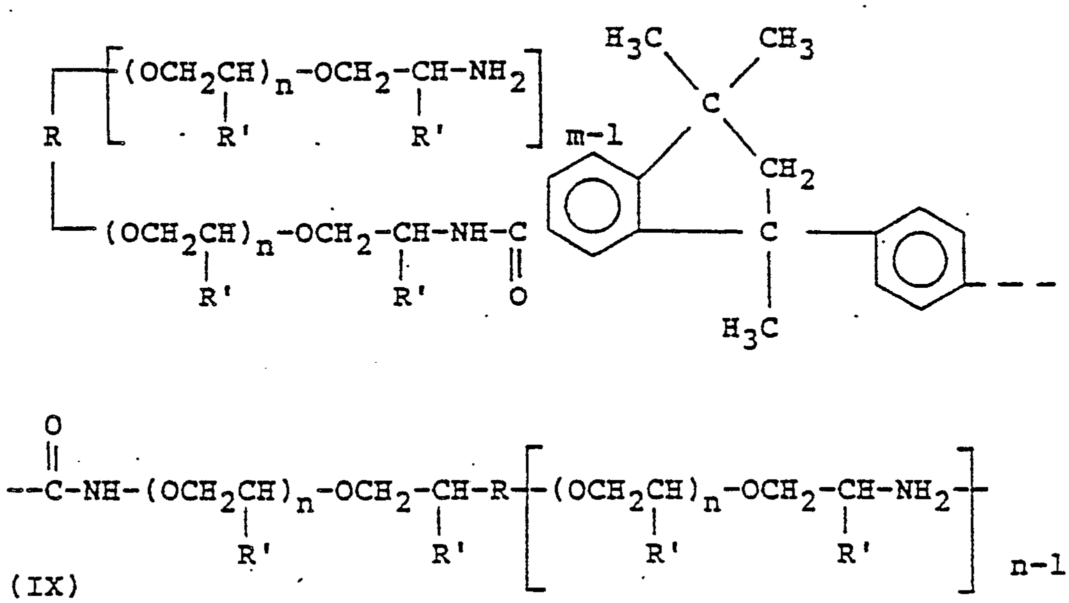

(IX)

wherein R, R′, m and n have the meanings given in claim 1,

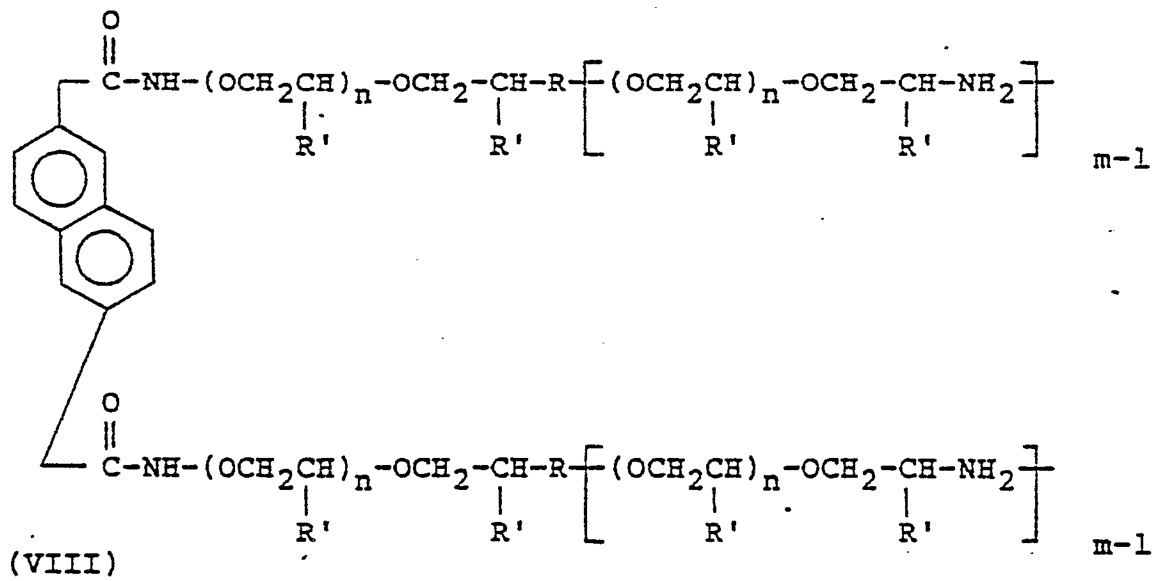

(VIII)

wherein R, R′, m and n have the meanings given in claim 1, or

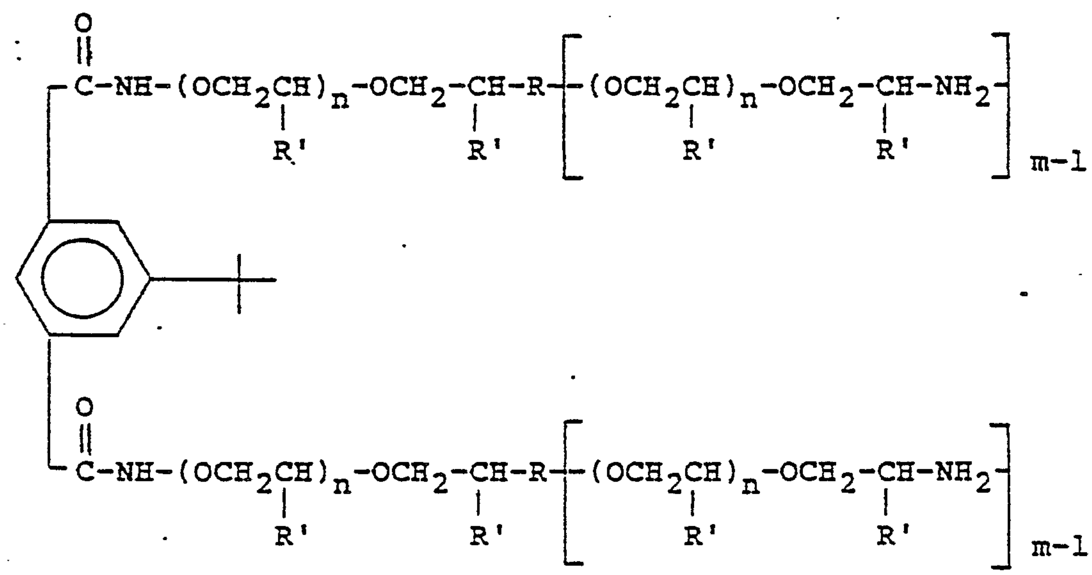

wherein R, R′, m and n have the meanings given in claim 1.

. A reaction product according to claim 2 characterised in that the carboxylic acid is trimesic acid, the polyamine is a polyoxyproplene diamine and the amidoamine has the formula:

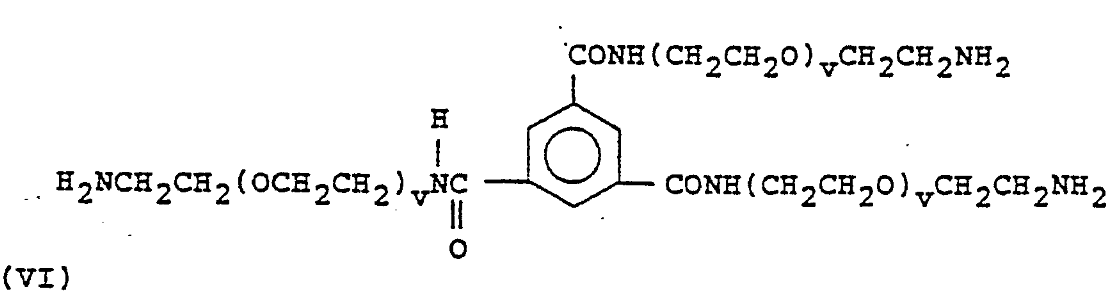

(VI)

wherein v is a number having a value of about 1-50.

8. A method of preparing an amidoamine characterised in that the method comprises reacting an aromatic di- or tricarboxylic acid, an anhydride or an ester thereof with at least 2 mole equivalents of a polyoxyalkylene polyamine, under reaction conditions including a temperature within the range of about 150° to about 250° C., autogenous pressure and a reaction time of about 0.5 to about 12 hours to thereby prepare a reaction product composed principally of an amidoamine formed by coupling each carboxyl group of said acid, ester or anhydride thereof through an amide group with a primary amine group of said polyalkylene polyamine, said polyoxyalkylene polyamine having the formula: defined in any of the preceding claims.

9. A reaction product according to Claim 1 characterised in that the product is obtained by reacting a polyoxypropylene and/or polyoxyethylene diamine with an aromatic dicarboxylic acid in molar proportions such that at least about 1.0 to 1.2 mole equivalents of an amine are provided in the reaction mixture for each mole equivalent of carboxylic acid present in the reaction mixture.

10. An epoxy resin composition which allows rapid curing even at ambient temperature to provide products having excellent coating properties, said composition characterised in that it comprises a mixture of:

1) uncured epoxy resin,
2) an alkyl substituted aromatic dicarboxylic acid; and
3) polyetheramine.

11. A composition according to claim 1 characterised in that the dicarboxylic acid is phenylindane, 1,1,3-trimethyl-3-phenylidane-4′, 5-dicarboxylic acid, and the condensation product of the dicarboxylic acid and polyetherpolyamine is of the formula:

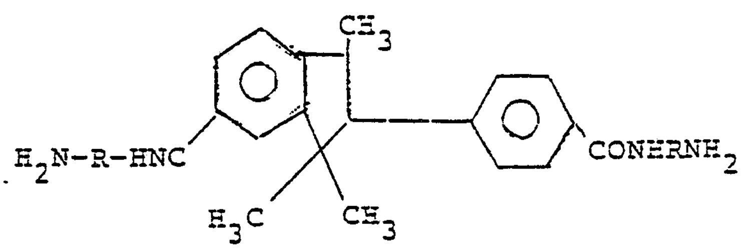

wherein R is a polyether segment from the group consisting of polyoxypropylene, polyoxyethylene of a combination of both, or
wherein the dicarboxylic acid is t-butylisophthalic and the amidoamine condensation product is of the formula:

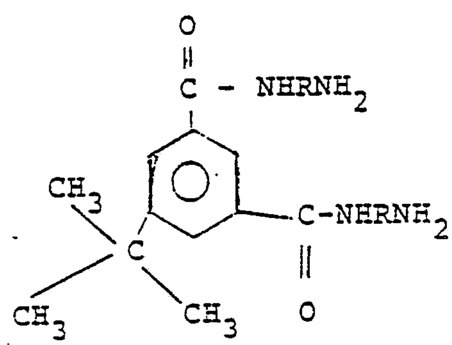

where R = a polyether segment consisting of polyoxypropylene or polyoxyethylene.

12. An epoxy resin composition which demonstrates rapid curing even at ambient temperature to provide materials useful in development of adhesive, coatings, encapsulants, laminates and composite fabrications, said composition characterised in that it comprises an amidoamine prepared from:

1) an alkyl substutited aromatic dicarboxylic acid consisting of phenylindane dicarboxylic and t-butylisophthalic acid; and a
2) polyetherpolyamine consisting of polyoxyethyleneamine and polyoxypropyleneamine; and
3) an epoxy resin where said amidoamine is the curing agent for the epoxy resin and is present in said mixture in an amount sufficient to provide about 0.8 to 1.2 amino groups per oxirane group.